# EUROPEAN PATENT SPECIFICATION

(11) **EP 1 341 119 B1**
(45) Date of publication and mention of the grant of the patent: **13.09.2006**
(21) Application number: 03003239.5
(22) Date of filing: 21.02.2003
(51) Int. Cl.: G06K 9/00, G06K 9/20

(54) **Iris recognition system**
System zur Iriserkennung
Système pour la reconnaisance de l'iris

(30) Priority: 21.02.2002 KR 2002009216
(43) Date of publication of application: 03.09.2003
(73) Proprietor: LG ELECTRONICS INC., Seoul (KR)
(72) Inventor: Lee, Won Hee, Dongjak-gu, Seoul (KR); Chae, Jang Jin, Goonpo-si, Gyeonggi-do (KR)
(74) Representative: Vetter, Ewald Otto

(56) References cited:
- EP-A- 0 589 191
- WO-A-01/20561
- US-A- 5 751 836
- US-A- 6 079 830

## Description

### BACKGROUND OF THE INVENTION

### 1. Field of the Invention

The present invention relates to an iris recognition system. In particular, the present invention relates to an eye position display and a method thereof, that is, where a user's eyes are supposed to be, through a cold mirror, as the user non-contactly focuses his/her eyes into the iris recognition system by standing within a distance where the system can recognize the user's iris. An iris recognition system according to the preamble of claim 1 is known for US 5,751,836. It provides several coloured LEDs which, in combination with a target pattern mask, generate visual angular alignment uses for the user. EP 0 589 191 A1 relates to a non-invasive glucose measurement method and apparatus.

### 2. Background of the Related Art

Well known to those skilled in the art, there have been a number of systems for security, crime prevention, and identity authentication, starting from the traditional contact cards system or the non-contact cards system, the fingerprint recognition system, and up to the iris recognition system. These all serve to authenticate a person's identification before allowing or denying the person's access to a particular place or data.

Among those, the iris recognition system is highly considered for its excellent recognition rate compared with the fingerprint recognition, and high accuracy. The iris recognition system authenticates a person by comparing a pre-registered iris data with a new iris data that has been prepared by photographing the person's iris images with a video camera and making characteristic iris patterns into data using an image process technique.

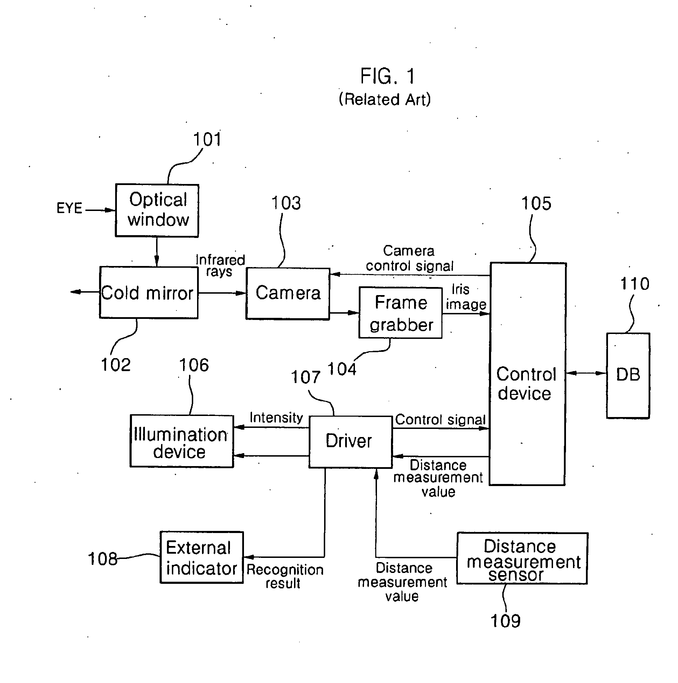

Fig. 1 is a schematic diagram of a general iris recognition system in a related art. With reference to Fig. 1, operation of the iris recognition system is now explained below.

When a user approaches the iris recognition system, a distance measurement sensor 109 measures the distance between the user to the system, and a control unit 105, having received the distance measurement value through a driver 107, decides whether the distance measurement value is within the operation limit.

If it turns out that the user is within the operation limit, a control signal is sent out to the driver 107 to extract the user's iris images.

The driver 107 sends an active signal to an external indicator 108 and lets the user know that the system is on. When the user puts his or her eyes on an optical axis of a camera 103 through an optical window 101, a cold mirror 102 blocks a visible ray and passes infrared rays.

Then the system indicates whether the iris of the user is supposed to be to make sure the user's eyes are properly positioned on the optical axis of the camera 103.

The control device 105 is provided with the distance value from the distance measurement sensor 109 to the user, and provided that the camera is an auto zoom focus camera, the control device 105, based on the distance measurement value, calculates zoom and focus values of the camera 103 and performs zoom in/zoom out and focusing control.

If the camera is a short focus lens, it gets much easier to photograph the user's iris image as long as the user is at the right position.

Later, the control device 105 photographs the iris image through the driver 107 in accordance with the distance measurement. The photographed iris image goes through a signal process at a frame grabber 104 suitable for the iris image analysis, and based on the pre-stored information about the processed iris image, the control device 105 performs iris recognition to authenticate the user.

The performance of this iris recognition system is heavily dependent on how fast and how accurately it can recognize the iris.

For that reason, the iris recognition system is typically mounted with a cold mirror for displaying the accurate position of the user's iris. Considering that the conventional iris recognition system makes the user come to the system and see whether his/her iris is accurately positioned at the optical axis of the camera, use of the cold mirror must be very convenient for faster and more accurate iris recognition.

Nevertheless, even the cold mirror gives rise to another problem called 'strong eye' phenomenon, which is brought forth because the position of the iris is simply reflected by a line drawn on the cold mirror or a LED (Light Emitting Diode) mounted inside the cold mirror focuses the iris.

The strong eye phenomenon occurs mainly because every person has different visions. For instance, suppose that the user sees his/her eyes on a 3cm x 3cm mirror from farther than 30cm. Although the user is supposed to focus by looking into his/her left eye (right eye) in the mirror with his left eye (right eye), in reality, the user looks into his/her right eye (left eye) using his/her left eye (right eye).

As such, the user could be certain that his/her iris has been properly focused after looking into the cold mirror by personally approaching the cold mirror, but due to the strong eye phenomenon, the user's eyes are often found away from the optical axis of the camera.

### SUMMARY OF THE INVENTION

An object of the invention is to solve at least the above problems and/or disadvantages and to provide at least the advantages described hereinafter.

Accordingly, one object of the present invention is to solve the foregoing problems by providing an iris recognition system having an eye position display and its method capable of recognizing a user's iris much faster and more accurately by guiding the user through an external indicator to stand within the distance where the user's iris can be recognized by the system and indicating through a cold mirror where the user needs to put his/her eyes.

The foregoing and other objects and advantages are realized by providing an iris recognition system, which includes: a board including a controller for controlling the emission of light; a light emitter (i.e. LED) for emitting a light from an illuminant housed in the board and uniformly scattering and equalizing the intensity of the emitted light; a lens unit for maintaining a focal length of the light from the LED, refracting and converging the light at a constant angle, and to display the position of a user's eye, passing the light at a predetermined portion only; and a reflector unit for reflecting every wavelength band that passed through the lens except for a wavelength of a predetermined light.

According to another aspect of the invention, a method of recognizing an iris includes the steps of: (1) pluralizing a distance between a user and an iris recognition system; (2) if the user approaches the iris recognition system, emitting a light from at least one light emitter (i.e. LED), and passing the light pass through a diffuser and a mask; (3) allowing the user to see the refracted light from the lens; (4) giving various instructions in accordance with a view angle of the user toward a reflector unit or a distance between the user and the iris recognition system; and (5) adjusting the distance from the user to the iris recognition system following the instructions manifested on the refractor unit.

According to another aspect of the invention, the second step includes the sub-steps of: emitting a light from at least one light emitter (i.e. LED) as the user approaches the iris recognition system; allowing the user to see a chip LED light when the user looks into the reflector unit, namely cold mirror, perpendicularly; and diffusing the LED light through a diffusion sheet and passing it through the mask.

According to another aspect of the invention, the third or fourth step includes the sub-steps of: passing the masked light through a tube for maintaining a focal length; collecting the light through the lens' refraction, thereby enabling the user to see the refractor with only one eye; and giving various instructions in accordance with a view angle of the user toward a reflector unit or a distance between the user and the iris recognition system.

Hence, the present invention can be very advantageously used for faster, more accurate iris recognition by giving an instruction to guide the user to come to the point where the user's iris is easily recognized by the iris recognition system and displaying through the cold mirror where the user is supposed to put his/her eye.

Additional advantages, objects, and features of the invention will be set forth in part in the description which follows and in part will become apparent to those having ordinary skill in the art upon examination of the following or may be learned from practice of the invention. The objects and advantages of the invention may be realized and attained as particularly pointed out in the appended claims.

### BRIEF DESCRIPTION OF THE DRAWINGS

The invention will be described in detail with reference to the following drawings in which like reference numerals refer to like elements wherein:

Fig. 1 is a schematic diagram of the structure of a general iris recognition system in a related art;

Fig. 2 is a schematic diagram of the structure of an eye position display of iris recognition system according to the present invention;

Fig. 3 is a block diagram illustrating the eye position display of the iris recognition system according to the present invention;

Fig. 4 is a perspective view of outside the iris recognition system according to the present invention;

Fig. 5 is a perspective view of inside the iris recognition system according to the present invention;

Figs. 6 and 7 diagrammatically explain the mechanism principles of the eye position display of the iris recognition system according to the present invention;

Fig. 8 illustrates some patterns shown on the eye position display of the iris recognition system according to a preferred embodiment of the present invention; and

Fig. 9 is a flow chart explaining the operation procedure of the iris recognition system according to the present invention.

### DETAILED DESCRIPTION OF PREFERRED EMBODIMENTS

The following detailed description will present an eye position display of the iris recognition system and a method thereof according to a preferred embodiment of the invention in reference to the accompanying drawings.

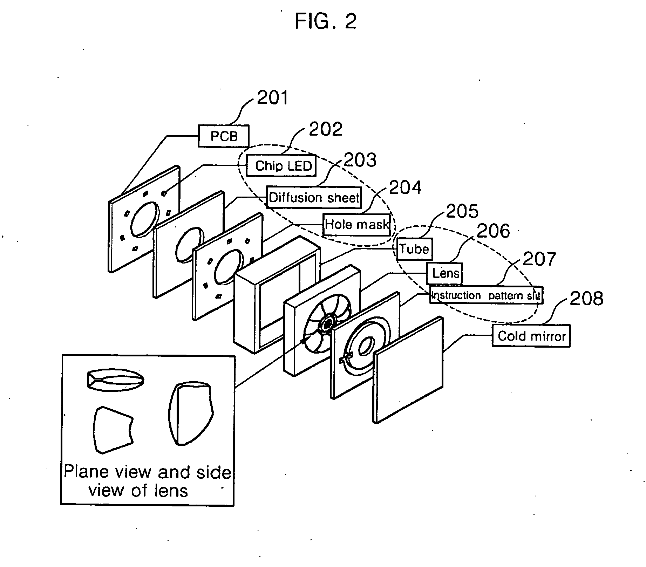

Fig. 2 is a schematic diagram of the structure of an eye position display of iris recognition system according to the present invention.

As depicted in the drawing, the eye position display includes PCB 201 mounted with a controller for controlling emission of light; chip LED 202 for emitting light, being mounted on the PCB 201; a diffusion sheet 203 for uniformly diffusing emitted light from the chip LED 202; a hole mask 204 for passing the uniformly diffused light from the diffusion sheet 203 only a predetermined region; a tube 205 operating as a spacer for the light having passed through the hole mask 204 to be focused; a lens 206 for converging the focused light at the tube 205; an instruction pattern slit 207 for passing the converged light in conformation to an instruction given; and a cold mirror 208 for exhibiting the light that passed through the instruction pattern slit 207.

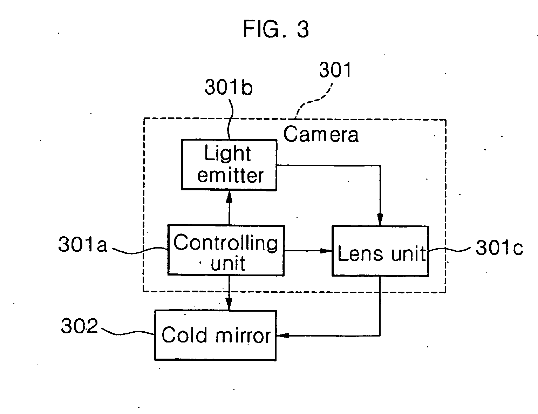

Fig. 3 is a block diagram illustrating the eye position display of the iris recognition system according to the present invention, the eye position display being installed inside the camera 103 of Fig. 1.

As shown in Fig. 3, the eye position display includes PCB 301a mounted with the controller for controlling the emission of light; a light emitter 301b for emitting light from LED included in the PCB and uniformly diffusing the emitted light, thereby making the light have uniform intensity; a lens 301c for maintaining a focal length of the emitted light provided by the light emitter (i.e. LED), refracting and converging the light at a constant angle, and passing the light through only a predetermined region; and a cold mirror 302 for refracting every visible ray having passed through the lens except for a wavelength of the emitted light from the LED and passing through infrared rays.

Preferably, the light emitter 301b includes the chip LED 202, the diffusion sheet 203, and the hole mask 204 shown in Fig. 2; and the lens unit 301c includes the tube 205, the lens 206, and the instruction pattern slit 207 shown in Fig. 2.

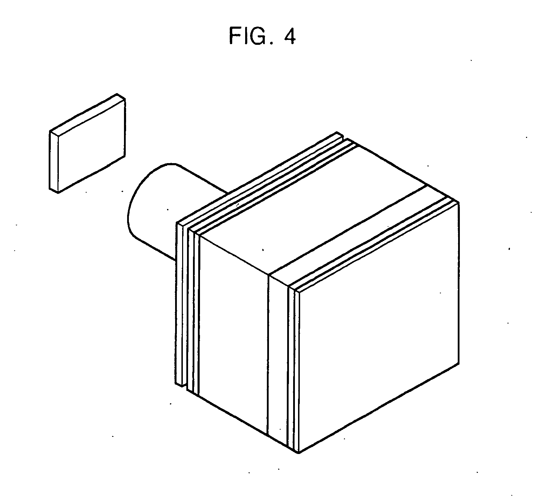

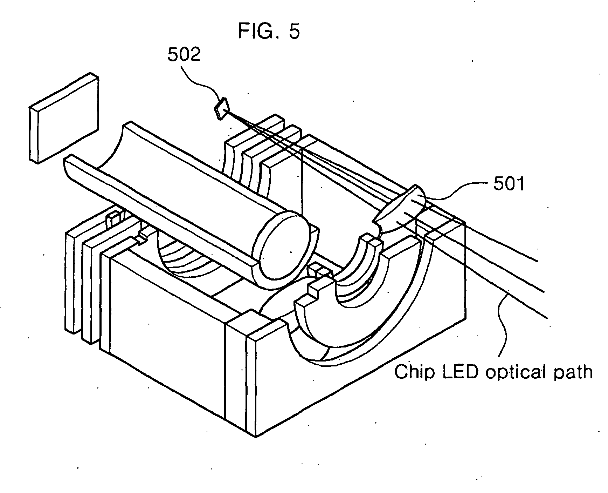

Fig. 4 is a perspective view of outside the iris recognition system according to the present invention, and Fig. 5 is a perspective view of inside the iris recognition system according to the present invention.

Now a preferred embodiment of the present invention will be described with reference to Figs. 3, 4, and 5.

The eye position display of the iris recognition system is installed in a camera recognition unit 103 of the system illustrated in Fig. 1. The PCB 201 is a circuit substrate including a controller for controlling lighting of each LED 202, and connected to a distance measurement sensor (refer to 109 of Fig. 1). When the user approaches the system, the PCB lights the chip LED 202 according to the distance measured by the distance measurement sensor.

The chip LED 202 indicates a plurality of infrared LED in circular form.

Once the chip LED 202 emits light, the diffusion sheet 203 diffuses the light uniformly and the hole mask 204 passes the light only through a masked region.

To explain further, when the light from the chip LED passes through the diffusion sheet, a half opaque sheet causing diffused reflection, particularly the masked part by the hole mask has a homogeneous or uniform intensity.

The diffusion sheet is made of paper functioning as a writing paper.

Also, what is meant by the masked part is that the light is illuminated only at an instruction part with a designated pattern, and the rest of the region is darkened.

In other words, when the LED is turned on, the light is usually the brightest at the center and gets darker at a peripheral portion. However, this changes when the mask is used. The light is divided into two parts, i.e. a uniformly bright region and a completely dark region.

Hence, when the user look at an indicator, he/she should come to a certain point to be able to see the light, and the user will see that the rest of the part is completely invisible.

After the light passes through the hole mask 204, it then goes through the tube. It is done so as to maintain the focal length, thereby focusing the light into the cold mirror 208.

More specifically, one of features of the lens is that it has a certain focal length, and the hole mask acts as one image, meaning that the user can see a clear image only when the distances among the hole mask, the lens, and the user's eye looking into the lens are well coordinated.

This is why the tube is employed as a spacer for adjusting the distances therebetween.

In other words, the hole mask and the lens are placed close to the focal length of the lens. When the user looks into the hole mask through the lens and moves his/her eye right to left or up and down by adjusting the distance between the hole mask and the lens, the motion region of the eye is determined.

Therefore, when the user's eye goes off the optical axis by a certain degree, the user cannot see the light of the hole mask.

Meanwhile, the light within the focal length from the tube 205 passes through the lens 206 and is converged until it is focused.

Following the shape of the LED mounted on the chip LED, each lens 206 is circular in order to converge the light from each LED.

Also, both ends of the lens are cut off for preventing refraction at the tangential parts where the lenses of each LED are connected. In this manner, a plurality of lenses can be connected to each other.

In more detail, every side (right, left, up, and down) of the lens is cut off.

The lens has a sphere shape on its front and rear part.

If seen from the front, the lens is always circular. Although there used to be much space between two lenses when the lenses are arrayed in conical shape, this is easily solved by cutting the circular shaped lenses to a fan-shape (sector form).

Every LED light converged by the lens 206 passes through the instruction pattern slit 207 and is shown on the cold mirror 208 after the slit pattern.

Therefore, the cold mirror 208 shows a circular-shaped band light to the user.

As aforementioned, the cold mirror 208 reflects all wavelength bands except for the wavelength of LED among visible rays that have passed through, and passes through infrared rays.

In short, when the user looks into the cold mirror, he/she should be able to see both the chip LED light coming through the lens and his/her own eye.

That is, the cold mirror should pass the LED light for the user to be able to see the LED light. Similarly, the light reflected from the user's eye by the peripheral light is reflected from the cold mirror and returns to the user's eye, whereby the user can see the light again.

To be short, the user sees his/her own eye through the cold mirror and at the same time the LED light behind the cold mirror.

As for the infrared ray region, there is operated an illumination device (not shown), originally installed for iris recognition, emits the infrared rays.

The infrared rays are reflected from the user's eye, pass through the cold mirror, and arrive at the camera.

Therefore, all wavelength bands except for the infrared rays, LED lights, and visible rays corresponding to LED lights are reflected.

The wavelength of light is expressed by nanometer. For instance, the wavelength of infrared rays is greater than 700nm, and that of visible rays is in the range from 450 to 670nm. If the wavelength of LED is 650 - 680nm, the cold mirror passes the wavelengths that exceed 650m and reflects wavelengths within the range from 450 to 649nm.

Such light is seen only when the user looks into the cold mirror 208 perpendicularly. This is done so as to prevent the 'strong eye' phenomenon. Basically, the 'strong eye' phenomenon occurs mainly because every person has different visions. For instance, suppose that the user sees his/her eyes on a 3cm x 3cm mirror from farther than 30cm. Although the user is supposed to focus by looking into his/her left eye (right eye) in the mirror with his left eye (right eye), in reality, the user looks into his/her right eye (left eye) using his/her left eye (right eye).

To prevent this phenomenon, the lens 206 should be able to collect the light to an angle that the user can see the light with only one eye.

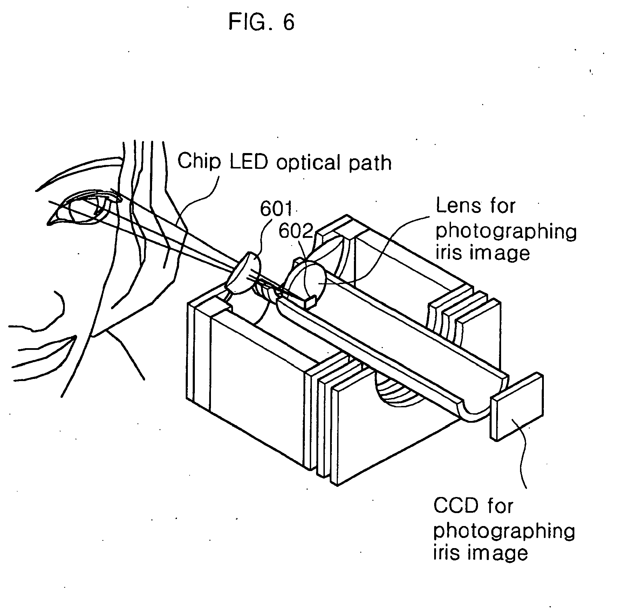

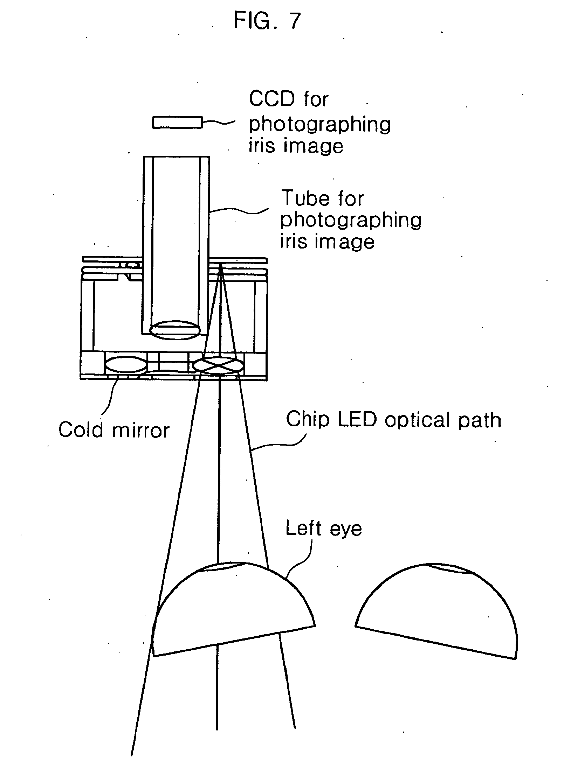

Figs. 6 and 7 diagrammatically explain the mechanism principles of the eye position display of the iris recognition system according to the present invention.

As illustrated in the drawings, the light from the chip LED 602 passes through the lens 601, and is refracted to a certain angle so that the user can see the light with his/her one eye only.

In this way, the user can see the light through the cold mirror, successfully avoiding the strong eye phenomenon.

Moreover, the user is provided with an instruction for position. This is possible by differentiating the pattern of the circular band shown on the cold mirror according to lighting of each LED.

Whether each LED should be turned on or not is determined in consideration with the distance between the iris recognition system and the user. The LED light guides the user to a proper distance from the system since the camera can obtain a clear image by adjusting its focal length only when the distance between the system and the user is as it should be.

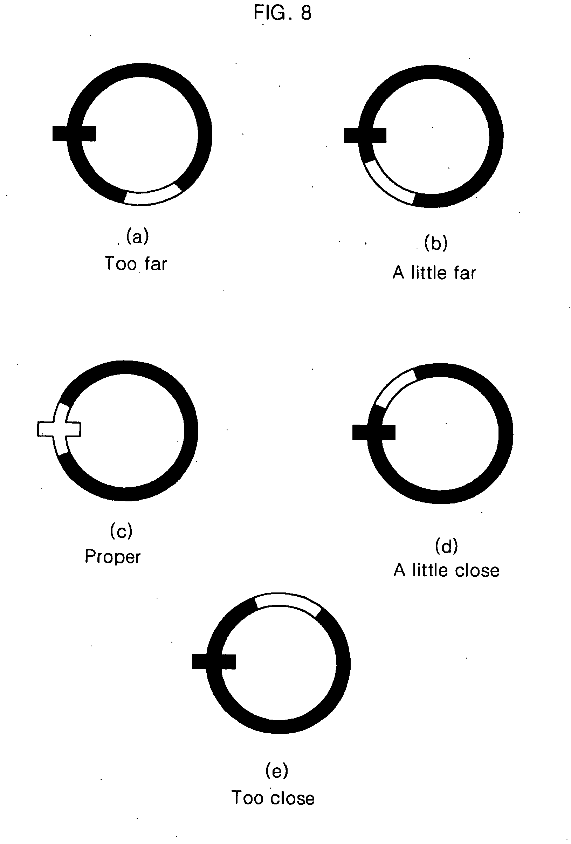

Fig. 8 illustrates some patterns shown on the eye position display of the iris recognition system according to a preferred embodiment of the present invention.

As seen from the drawing, the circular band light looks different depending on the distance between the iris recognition system and the user.

For instance, if the distance is correct, (c) shows. However, if the distance is rather far, (A) or (B) shows. Lastly, if the distance is close, (D) or (E) shows.

The distance indication patterns can be designed in diverse ways, as long as the above principles are applied thereto.

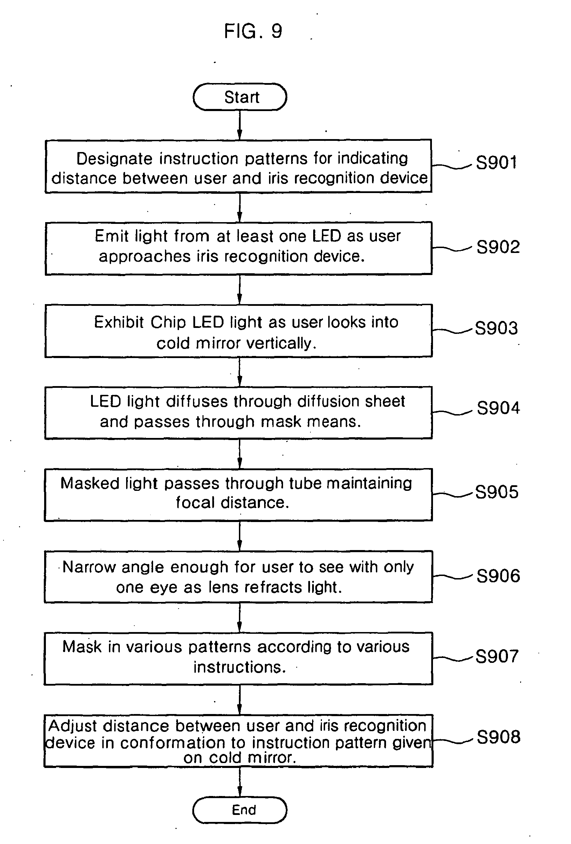

Fig. 9 is a flow chart explaining the operation procedure of the iris recognition system according to the present invention.

First of all, there are more than two different instruction patterns for indicating the distance between the iris recognition system and the user (S901).

As the user approaches the iris recognition system, at least one LED emits light (S902).

The user can see the chip LED light only when he/she looks into the reflector unit, namely the cold mirror, perpendicularly (S903).

The LED light is diffused through the diffusion sheet and passes through the mask means, namely hole mask (S904).

The masked light passes through the tube for maintaining its focal distance, and the lens refracts the light, enabling the user can see the light with his/her one eye only (S905 and S906).

In accordance with the view angle of the user toward the cold mirror or the distance between the user and the iris recognition system, various instruction patterns show on the cold mirror via the instruction pattern slit (S907).

The user adjusts the distance to the iris recognition system following the instruction pattern shown on the reflector unit (S908).

The foregoing embodiments and advantages are merely exemplary and are not to be construed as limiting the present invention which is defined by the appended claims. The present teaching can be readily applied to other types of apparatuses. The description of the present invention is intended to be illustrative, and not to limit the scope of the claims. Many alternatives, modifications, and variations will be apparent to those skilled in the art.

## Claims

1. An iris recognition system, comprising:
a light emitter (202, 203, 204; 301b; 502; 602) for emitting a light and uniformly scattering the emitted light;
**characterized by** a board (201) including a controller for controlling emission of light; the light emitter being mounted on the board and equalizing the intensity of the emitted light;
a lens unit (205, 206, 207; 301c; 501; 601) for maintaining a focal length of the emitted light from the light emitter (202, 203, 204; 301b; 502; 602), refracting and converging the light at a constant angle, and passing the light through a predetermined portion (207) only so as to display a position where a user's eye is or should be;
wherein the light emitter comprises a plurality of light emitting means which are selectively lighted according to a measured distance between the iris recognition system and a user, the light emitted by which being masked by said predetermined portion so as to achieve various masked instruction patterns, each indicating a distance to the user; and
a reflector unit (208; 302) for reflecting every wavelength band that passed through the lens unit (205, 206, 207; 301c; 501; 601) except for a wavelength of a predetermined light.

2. The system according to claim 1, wherein the light emitter (202, 203, 204; 301b; 502; 602) comprised a chip LED (202; 502; 602) including a plurality of infrared ray LEDs and a diffusion sheet (203) for uniformly diffusing the emitted light.

3. The system according to claim 2, wherein the lens unit (205, 206, 207; 301c; 501; 601) consists of or comprises circular-shaped lenses (206; 501; 601) following the form of LED mounted on the chip LED (202; 502; 602) to be able to converge each LED light.

4. The system according to claim 3, wherein both ends of each LED lens (206; 501; 601) are cut off to prevent refraction at tangential parts where the lenses (206; 501; 601) of each LED are connected, whereby a plurality of lenses can be connected to each other.

5. The system according to at least one of the preceding claims, wherein the light emitter (202, 203, 204; 301b; 502; 602) further comprises a hole mask (204) for passing the light only through a predetermined part and equalizing intensity of the light passed through.

6. The system according to at least one of the preceding claims, wherein the lens unit (205, 206, 207; 301c; 501; 601) further comprises a tube (205) for keeping the light from the light emitter (202, 203, 204; 301b; 502; 602) at a predetermined distance to focus the light.

7. The system according to at least one of claims 2 to 6, wherein predetermined portion of the lens unit (205, 206, 207; 301c; 501; 601) is an instruction slit (207) for manifesting every converged LED light in form of said various masked patterns.

8. The system according to claim 7, wherein the masked patterns are shown on the reflector unit, namely cold mirror (208; 302).

9. The system according to at least one of the preceding claims, wherein the lens unit (205, 206, 207; 301c; 501; 601) for converging the light comprises a lens (206; 501; 601) for performing at least one of refraction and convergence of the light at a predetermined angle to enable the user to see the light with one eye only.

10. The system according to at least one of the preceding claims, wherein the user can see an emitted light from the light emitter (202, 203, 204; 301b; 502; 602), namely LED, only when the user sees the reflector unit, namely cold mirror (208; 302), at a predetermined angle.

11. The system according to at least one of the preceding claims, wherein the lens unit (205, 206, 207; 301c; 501; 601) comprises a reflector unit (208; 302) for reflecting every wavelength band except for a wavelength of the light emitted from the LED among visible rays of the light that passed through the lens (206; 501; 502), and passing infrared rays.

12. A method of recognizing an iris, comprising :
(1) measuring a distance between a user and an iris recognition system;
(2) if the user approaches the iris recognition system, emitting a light from at least one light emitter (202; 301b; 502; 602), and passing the light through a diffuser (203) and a mask (204); wherein the light emitter comprises a plurality of light emitting means which are selectively lighted according to the measured distance of the user, and the light emitted by which is masked (207) so as to achieve various masked instruction patterns each indicating a distance to the user;
(3) allowing the user to see the refracted light from the lens (206; 301c; 501; 601);
(4) showing said various instruction patterns in accordance with a view angle of the user toward a reflector unit (208; 302) and a distance between the user and the iris recognition system for
(5) guiding the user to a proper distance from the iris recognition system following the instructions manifested on the reflector unit (208; 302).

13. The method according to claim 12, where the second step comprises the sub-steps of:
emitting a light from at least one light emitter (202; 301b; 502; 602) as the user approaches the iris recognition system;
displaying a chip LED light only when the user looks into the reflector unit, namely cold mirror (208; 302), perpendicularly; and
diffusing the LED light through a diffusion sheet (203) and passing the same through the mask (204).

14. The method according to claim 12 or 13, wherein the third or fourth step comprises the sub-steps of:
passing the masked light through a tube (205) for maintaining a focal length;
collecting the light through the lens' refraction to enable the user to see the refracted light with only one eye .

## Patentansprüche

1. Iriserkennungssystem, aufweisend:
einen Lichtemitter (202, 203, 204; 301b; 502; 602) zum Emittieren eines Lichts und gleichmäßigen Streuen des emittierten Lichts;
**gekennzeichnet durch** eine Platte (201), welche eine Steuereinrichtung zur Steuerung der Lichtemission aufweist; wobei der Lichtemitter auf der Platte montiert ist und die Intensität des emittierten Lichts ausgleicht;
eine Linseneinheit (205, 206, 207; 301c; 501; 601) zur Aufrechterhaltung einer Brennweite des vom Lichtemitter (202, 203, 204; 301b; 502; 602) emittierten Lichts, zum Brechen und Konvergieren des Lichts unter einem konstanten Winkel und zum Hindurchleiten des Lichts **durch** einen vorbestimmten Teil (207) nur um eine Position anzuzeigen, wo das Auge eines Benutzers ist oder sein sollte;
wobei der Lichtemitter eine Mehrzahl von Lichtemissionsmitteln aufweist, welche selektiv beleuchtet werden entsprechend einem gemessenen Abstand zwischen dem Iriserkennungssystem und einem Benutzer, wobei das von diesen emittierte Licht **durch** den vorbestimmten Teil maskiert wird, um so verschiedene maskierte Instruktionsmuster zu erzielen, welche jeweils einen Abstand zum Benutzer angeben; und
eine Reflektoreinheit (208; 302) zum Reflektieren jedes Wellenlängenbandes, das **durch** die Linseneinheit (205, 206, 207; 301c; 501; 601) hindurchgelangt, mit Ausnahme einer Wellenlänge eines vorbestimmten Lichts.

2. System nach Anspruch 1, wobei der Lichtemitter (202, 203, 204; 301b; 502; 602) eine Chip-LED (202; 502; 602) aufweist, die eine Mehrzahl von Infrarotstrahlen-LEDs und einen Diffusionsbogen (203) zum gleichmäßigen Streuen des emittierten Lichts aufweist.

3. System nach Anspruch 2, wobei die Linseneinheit (205, 206, 207; 301c; 501; 601) aus kreisförmig geformten Linsen (206; 501; 601) besteht oder diese aufweist, welche der Form der LED folgen, die auf der Chip-LED (202; 502; 602) montiert ist, um in der Lage zu sein, jedes LED-Licht zu konvergieren.

4. System nach Anspruch 3, wobei beide Enden jeder LED-Linse (206; 501; 601) abgeschnitten sind, um eine Brechung an tangentialen Teilen, wo die Linsen (206; 501; 601) jeder LED verbunden sind, zu verhindern, wobei eine Mehrzahl von Linsen miteinander verbunden werden kann.

5. System nach mindestens einem der vorhergehenden Ansprüche, wobei der Lichtemitter (202, 203, 204; 301b; 502; 602) ferner eine Lochmaske (204) aufweist, um das Licht nur durch einen vorbestimmten Teil passieren zu lassen und die Intensität des hindurchgeleiteten Lichts auszugleichen.

6. System nach mindestens einem der vorhergehenden Ansprüche, wobei die Linseneinheit (205, 206, 207; 301c; 501; 601) ferner eine Röhre (205) aufweist, um das Licht vom Lichtemitter (202, 203, 204; 301b, 502; 602) auf einem vorbestimmten Abstand zu halten, um das Licht zu fokussieren.

7. System nach mindestens einem der Ansprüche 2 bis 6, wobei der vorbestimmte Teil der Linseneinheit (205, 206, 207; 301c; 501; 601) ein Instruktionsschlitz (207) ist, um jedes konvergierte LED-Licht in Form der verschiedenen maskierten Muster zu manifestieren.

8. System nach Anspruch 7, wobei die maskierten Muster auf der Reflektoreinheit, nämlich dem Kaltlichtspiegel (208; 302), dargestellt sind.

9. System nach mindestens einem der vorhergehenden Ansprüche, wobei die Linseneinheit (205, 206, 207; 301c; 501; 601) zum Konvergieren des Lichts eine Linse (206; 501; 601) aufweist, um mindestens eines von Brechen und Konvergieren des Lichts unter einem vorbestimmten Winkel durchzuführen, um es dem Benutzer zu ermöglichen, das Licht mit nur einem Auge zu sehen.

10. System nach mindestens einem der vorhergehenden Ansprüche, wobei der Benutzer ein vom Lichtemitter (202, 203, 204; 301b; 502; 602), nämlich LED, emittiertes Licht nur sehen kann, wenn der Benutzer die Reflektoreinheit, nämlich den Kaltlichtspiegel (208; 302), unter einem vorbestimmten Winkel sieht.

11. System nach mindestens einem der vorhergehenden Ansprüche, wobei die Linseneinheit (205, 206, 207; 301c; 501; 601) eine Reflektoreinheit (208; 302) zum Reflektieren jedes Wellenlängenbandes aufweist mit Ausnahme einer Wellenlänge des von der LED emittierten Lichts unter den sichtbaren Strahlen des Lichts, das durch die Linse (206; 501; 502) hindurchgelangt, und hindurchgehende Infrarotstrahlen.

12. Verfahren zur Erkennung einer Iris, aufweisend:
(1) Messen eines Abstandes zwischen einem Benutzer und einem Iriserkennungssystem;
(2) wenn sich der Benutzer dem Iriserkennungssystem nähert, Emittieren eines Lichts von mindestens einem Lichtemitter (202; 301b; 502; 602) und Hindurchleiten des Lichts durch einen Diffusor (203) und eine Maske (204); wobei der Lichtemitter eine Mehrzahl von Lichtemissionsmitteln aufweist, welche selektiv beleuchtet werden gemäß dem gemessenen Abstand des Benutzers und dem emittierten Licht, welches maskiert (207) ist, um so verschiedene maskierte Instruktionsmuster zu erzielen, welche je einen Abstand zum Benutzer angeben;
(3) es dem Benutzer ermöglichen, das gebrochene Licht von der Linse (206; 301c; 501; 601) zu sehen;
(4) Anzeigen der verschiedenen Instruktionsmuster gemäß einem Blickwinkel des Benutzers zu einer Reflektoreinheit (208; 302) hin und einem Abstand zwischen dem Benutzer und dem Iriserkennungssystem zum
(5) Führen des Benutzers zu einem richtigen Abstand von dem Iriserkennungssystem, den auf der Reflektoreinheit (208; 302) manifestierten Instruktionen folgend.

13. Verfahren nach Anspruch 12, wobei der zweite Schritt die Unterschritte aufweist:
Emittieren eines Lichts von mindestens einem Lichtemitter (202; 301b; 502; 602), wenn sich der Benutzer dem Iriserkennungssystem nähert;
Anzeigen eines Chip-LED-Lichts nur wenn der Benutzer senkrecht in die Reflektoreinheit, nämlich den Kaltlichtspiegel (208; 302) blickt; und
Streuen des LED-Lichts durch einen Diffusionsbogen (203) und Hindurchleiten desselben durch die Maske (204).

14. Verfahren nach Anspruch 12 oder 13, wobei der dritte oder vierte Schritt die Unterschritte aufweist:
Hindurchleiten des maskierten Lichts durch eine Röhre (205), um eine Brennweite aufrechtzuerhalten;
Sammeln des Lichts durch die Linsenrefraktion, um es dem Benutzer zu ermöglichen, das gebrochene Licht mit nur einem Auge zu sehen.

## Revendications

1. Système de reconnaissance de l'iris, comprenant :
un émetteur de lumière (202, 203, 204 ; 301b ; 502 ; 602) destiné à émettre une lumière et à diffuser uniformément la lumière émise,
**caractérisé par** une carte (201) comprenant un contrôleur destiné à commander l'émission de lumière, l'émetteur de lumière étant monté sur la carte et égalisant l'intensité de la lumière émise,
une unité de lentille (205, 206, 207 ; 301c, 501 ; 601) destinée à maintenir une distance focale de la lumière émise depuis l'émetteur de lumière (202, 203, 204 ; 301b ; 502 ; 602), à réfracter et à faire converger la lumière à un angle constant, et à faire passer la lumière au travers d'une partie prédéterminée (207) seulement de manière à afficher une position où se trouve ou devrait se trouver un oeil d'un utilisateur,
dans lequel l'émetteur de lumière comprend une pluralité de moyens d'émission de lumière qui sont sélectivement amenés à émettre de la lumière selon une distance mesurée entre le système de reconnaissance de l'iris et un utilisateur, la lumière émise par celui-ci étant masquée par ladite partie prédéterminée de manière à obtenir divers motifs d'instructions masqués indiquant chacun une distance à l'utilisateur, et
une unité de réflecteur (208 ; 302) destinée à réfléchir chaque bande de longueur d'onde qui passe au travers de l'unité de lentille (205, 206, 207 ; 301c, 501 ; 601) à l'exception d'une longueur d'onde d'une lumière prédéterminée.

2. Système selon la revendication 1, dans lequel l'émetteur de lumière (202, 203, 204 ; 301b ; 502 ; 602) est constitué d'une diode électroluminescente pastille (202 ; 502; 602) comprenant une pluralité de diodes électroluminescentes à rayon infrarouge et une feuille de diffusion (203) destinée à diffuser uniformément la lumière émise.

3. Système selon la revendication 2, dans lequel l'unité de lentille (205, 206, 207 ; 301c, 501 ; 601) est constituée ou comprend des lentilles en forme de cercle (206 ; 501 ; 601) suivant la forme de la diode électroluminescente montée sur la diode électroluminescente pastille (202 ; 502 ; 602) afin de pouvoir faire converger la lumière de chaque diode électroluminescente.

4. Système selon la revendication 3, dans lequel les deux extrémités de chaque lentille de diode électroluminescente (206 ; 501 ; 601) sont découpées afin d'empêcher une réfraction au niveau de parties tangentielles où les lentilles (206 ; 501 ; 601) de chaque diode électroluminescente sont connectées, grâce à quoi une pluralité de lentilles peuvent être connectées les unes aux autres.

5. Système selon au moins une des revendications précédentes, dans lequel l'émetteur de lumière (202, 203, 204 ; 301b ; 502 ; 602) comprend en outre un masque à trou (204) destiné à faire passer la lumière seulement au travers d'une partie prédéterminée et à égaliser l'intensité de la lumière passant au travers.

6. Système selon au moins l'une des revendications précédentes, dans lequel l'unité de lentille (205, 206, 207 ; 301c, 501 ; 601) comprend en outre (205) destiné à empêcher la lumière provenant de l'émetteur de lumière (202, 203, 204 ; 301b ; 502 ; 602) à une distance prédéterminée de se focaliser.

7. Système selon au moins l'une des revendications 2 à 6, dans lequel la partie prédéterminée de l'unité de lentille (205, 206, 207 ; 301c, 501 ; 601) est une fente d'instruction (207) destinée à manifester la lumière de chaque diode électroluminescente qui a convergé sous la forme desdits divers motifs masqués.

8. Système selon la revendication 7, dans lequel les motifs masqués sont représentés sur l'unité de réflecteur, à savoir un miroir froid (208, 302).

9. Système selon au moins l'une des revendications précédentes, dans lequel l'unité de lentille (205, 206, 207 ; 301c, 501 ; 601) destinée à faire converger la lumière comprend une lentille (206 ; 501 ; 601) destinée à exécuter au moins une réfraction et une convergence de la lumière à un angle prédéterminé pour permettre à l'utilisateur de voir la lumière avec un oeil seulement.

10. Système selon au moins l'une des revendications précédentes, dans lequel l'utilisateur peut voir une lumière émise à partir de l'émetteur de lumière (202, 203, 204 ; 301b ; 502 ; 602), à savoir une diode électroluminescente, seulement lorsque l'utilisateur voit l'unité de réflecteur, à savoir un miroir froid (208 ; 302), à un angle prédéterminé.

11. Système selon au moins l'une des revendications précédentes, dans lequel l'unité de lentille (205, 206, 207 ; 301c, 501 ; 601) comprend une unité de réflecteur (208 ; 302) destinée à réfléchir chaque bande de longueur d'onde à l'exception d'une longueur d'onde de la lumière émise à partir de la diode électroluminescente parmi les rayonnements du visible de la lumière qui passent au travers de la lentille (206 ; 501 ; 202), et des rayons infrarouges qui passent.

12. Procédé de reconnaissance de l'iris comprenant :
(1) la mesure d'une distance entre un utilisateur et un système de reconnaissance de l'iris,
(2) si l'utilisateur s'approche du système de reconnaissance de l'iris, émettre une lumière à partir d'au moins un émetteur de lumière (202 ; 301b ; 502 ; 602), et le fait de faire passer la lumière au travers d'un diffuseur (203) et d'un masque (204), où l'émetteur de lumière comprend une pluralité de moyens d'émission de lumière qui sont sélectivement amenés à éclairer conformément à la distance mesurée de l'utilisateur, et la lumière émise par celui-ci est masquée (207) de manière à obtenir divers motifs d'instruction masqués, indiquant chacun une distance à l'utilisateur,
(3) permettre à l'utilisateur de voir la lumière réfractée provenant de la lentille (206 ; 301c ; 501 ; 601),
(4) présenter divers motifs d'instruction conformément à un angle de vue de l'utilisateur en direction de l'unité de réflecteur (208 ; 308) et une distance entre l'utilisateur et le système de reconnaissance de l'iris pour
(5) guider l'utilisateur à une distance appropriée du système de reconnaissance de l'iris en suivant les instructions manifestées sur l'unité de réflecteur (208 ; 302).

13. Procédé selon la revendication 12, où la seconde étape comprend les sous-étapes consistant à :
émettre de la lumière à partir d'au moins un émetteur de lumière (202, ; 301b ; 502 ; 602) lorsque l'utilisateur s'approche du système de reconnaissance de l'iris,
afficher une lumière de diode électroluminescente pastille seulement lorsque l'utilisateur regarde à l'intérieur de l'unité de réflecteur, à savoir un miroir froid (208; 302), perpendiculairement, et
diffuser la lumière de diode électroluminescente au travers d'une feuille de diffusion (203) et faire passer celle-ci au travers du masque (204).

14. Procédé selon la revendication 12 ou 13, dans lequel la troisième ou quatrième étape comprend les sous-étapes consistant à :
faire passer la lumière qui est passée par le masque au travers d'un tube (205) destiné à maintenir une certaine distance focale,
collecter la lumière par l'intermédiaire de la réfraction de lentille pour permettre à l'utilisateur de voir la lumière réfractée avec seulement un oeil.
